Europäisches Patentamt

European Patent Office

Office européen des brevets

(11) Publication number: **0 080 382**
**B1**

# EUROPEAN PATENT SPECIFICATION

(12)

(45) Date of publication of patent specification: **10.08.88**

(21) Application number: **82306269.0**

(22) Date of filing: **24.11.82**

(51) Int. Cl.⁴: **D 04 H 1/56,** A 61 L 15/00 //
A47L13/16

(54) Microfibre web product.

(30) Priority: **24.11.81 GB 8135331**

(43) Date of publication of application:
**01.06.83 Bulletin 83/22**

(45) Publication of the grant of the patent:
**10.08.88 Bulletin 88/32**

(84) Designated Contracting States:
**AT BE CH DE FR GB IT LI LU NL**

(56) References cited:
**DE-B-1 253 117**
**FR-A-1 527 536**
**US-A-2 464 301**
**US-A-2 988 469**
**US-A-3 016 599**
**US-A-4 100 324**
**US-A-4 103 058**
**US-A-4 186 165**

(73) Proprietor: **KIMBERLY-CLARK LIMITED**
**Larkfield**
**Maidstone Kent, ME20 7PS (GB)**

(72) Inventor: **Minto, Mansoor Ahmad, Dr.**
**2 Jerome Road**
**Larkfield Kent (GB)**
Inventor: **Storey, Dennis Graham**
**1a The Landway**
**Bearsted Kent (GB)**
Inventor: **Owen, Geoffrey Robert, Dr.**
**55 Cherry Orchard**
**Ditton Kent (GB)**

(74) Representative: **Allen, Oliver John Richard et al**
**Lloyd Wise, Tregear & Co. Norman House**
**105-109 Strand**
**London, WC2R 0AE (GB)**

EP 0 080 382 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to non-woven fabrics and in particular to those comprising a matrix of melt blown polymer fibres and a method of producing these.

Fabric made from melt blown polymer fibres and absorbent wood pulp fibre material is known from US—A—4100324.

In accordance with one aspect of the invention, a fluid retentive web comprises entangled meltblown polymer microfibres having an average fibre diameter greater than about 1 micron and absorbent material held within the web, characterised in that the material comprises particles of super absorbent (e.g. synthetic organic polymer, modified starch, cellulose, alginate or the like) distributed substantially individually and spaced throughout the web.

In accordance with another aspect of the invention, a method of making. a fluid retentive non-woven web comprises extruding molten polymeric material in such a way as to produce a stream of meltblown polymeric microfibres having an average fibre diameter greater than 1 micron, directing absorbent material onto the stream of fibres, and then forming or consolidating the fibres into a web characterised in that particles of super absorbent (e.g. synthetic organic polymer, modified starch, cellulose, alginate or the like) are directed into the stream of meltblown fibres to be distributed substantially individually and spaced throughout the web, and in that an electrostatic charge is applied to the particles of super absorbent.

The particles are preferably blown onto the stream of particles shortly after the fibres leave an extrusion nozzle and the particles may be given an electrostatic charge prior to contacting the fibres which helps to separate the particles in the web.

Other fibres such as wood pump fibres or staple textile fibres (e.g. cotton) may also be introduced preferably simultaneously with the absorbent particles.

Preferably the fibres of the web have a diameter between 1 and 50 microns, with most microfibres preferably less than 10 microns. The fibres may, for example, be of polyester, polypropylene or nylon.

A wetting agent may be added to improve the water absorbency properties.

The particles may be relatively small, e.g. 1 micron or less up to 100 microns or larger and may be incorporated as individual particles or as clusters.

Particulate super absorbent material when added to the molten melt blown microfibres produces a web with significant unexpected benefits. The resultant web utilises the excellent wicking properties of melt blown microfibres, i.e. the high capillary attraction present between microfibres, to rapidly convey fluid to the finely dispersed individual super absorbent particles. . This isolation of the individual particles can inbibe fluid without substantial interference from gel blocking. Isolation may, for example, be produced by giving the particles an electrostatic charge before feeding them into the stream of fibres.

Gel blocking occurs when a mass of super absorbent swells upon imbibation. This swelling acts to substantially diminish the size of the capillaries in the super absorbent mass and may, in fact, close them. While other attempts at providing maximum surface per weight have been utilised, none have utilised individual isolated super absorbent particles in combination with melt blown microfibres to provide this superior wicking in combination with absorbent efficiency.

During imbibation the entire microfibre super absorbent composite swells but there is still isolation of individual particles of super absorbent. Therefore, while swelling occurs, gel blocking does not. This is true even at high levels of super absorbent addition to the composite and in fact a proportional increase in capacity and horizontal wicking is observed.

The particles of super absorbent material have relatively large diameter compared to the diameter of the individual microfibres and thus tend to be trapped within a network of the fibres and therefore little surface tack of the fibres is needed to maintain the super absorbent particles in place.

Webs with super absorbent particles may, for example, be used in sanitary napkins, diapers, incontinence pads or the like.

In order to increase its strength, the web in accordance with the invention may be hot calendered or embossed with heated patterned bonding rolls. The fabric may also be perforated as described and claimed in EP—A—0080383. This further improves the absorbency and wiping properties of the fabric.

The invention will now be further described by way of example with reference to the accompanying drawings in which:—

Figure 1 is a partly schematic side elevation of an apparatus for producing fabrics according to the present invention;

Figure 2 is a plan view of a fragment of fabric according to the present invention which has been embossed;

Figure 3 is a cross-section of one form of embossment in the fabric of Figure 1;

Figure 4 is an electron microscope photograph of a fabric having cellulose sponge particles;

Figure 5 is a diagrammatic illustration of an alternative apparatus for producing webs in accordance with the inventioon;

Figure 6 is a graph comparing saturated capacity of super absorbent composites against a tissue laminated super absorbent versus applied pressure;

Figure 7 is a graph showing the volume (thickness) of microfibre super absorbent composites versus applied pressure;

Figure 8 is a graph showing the volume (thickness) of wood fluff absorbent versus applied pressure;

Figure 9 is a graph showing the wickening of super absorbent composites versus percent concentration, and

Figure 10 is an electron microscope photograph of fabric with super absorbent particles.

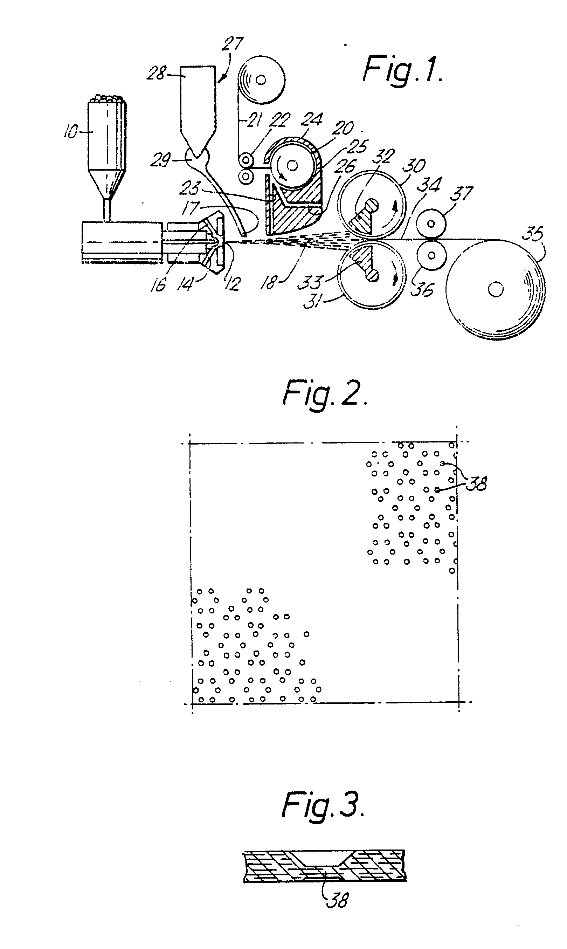

Referring to Figure 1, discontinuous thermoplastic polymeric material from a hopper 10 is heated and then caused to flow through nozzle 12 whilst being subjected to air jets through nozzles 14, 16 which produces a final stream 18 containing discontinuous microfibres of the polymeric material. This is known as melt-blowing and the technique is further described in an article entitled "Superfine Thermoplastic Fibres" appearing in Industrial and Engineering Chemistry, Vol. 48, No. 8, pp 1342—1346 which describes work done at the Naval Research Laboratories in Washington D.C. Also see Naval Research Laboratory Report No. 11437 dated 15th April 1954, U.S. Patent No. 3,676,242 and U.S. Patent No. 4,100,324 issued to Anderson et al.

The process described as follows with reference to figure 1 is claimed in the divisional application EP—A—156160.

The apparatus shown in Figure 1 is generally the same as described in U.S. Patent No. 4,100,324 with the exception of two particular features which will be described hereinafter and the subject matter of that patent is to be considered as being included in the present specification and will not be further described. The subject matter of U.S. Patent No. 3,793,678 entitled "Pulp Picking Apparatus with Improved Fibre Forming Duct" is also to be considered as being included in the present specification insofar as the picker roll 20 and feed 21 to 26 are concerned, as also described in U.S. Patent No. 4,100,324.

The picker roll 20 and associated feed 21 to 26 are an optional feature of the apparatus of Figure 1 and are provided to enable the introduction of fibrous material into the web of the invention if this is required.

The picker device comprises a conventional picker roll 20 having picking teeth for divellicating pulp sheets 21 into individual fibres. The pulp sheets 21 are fed radially, i.e., along a picker roll radius, to the picker roll 20 by means of rolls 22. As the teeth on the picker roll 20 divellicate the pulp sheets 21 into individual fibres, the resulting separated fibres are conveyed downwardly toward the primary air stream through a forming nozzle or duct 23. A housing 24 encloses the picker roll 20 and provides a passage 25 between the housing 24 and the picker roll surface. Process air is supplied to the picker roll in the passage 25 via duct 26 in sufficient quantity to serve as a medium for conveying the fibres through the forming duct 23 at a velocity approaching that of the picker teeth. The air may be supplied by any conventional means as, for example, a blower.

It has been found that, in order to avoid fibre floccing, the individual fibres should be conveyed through the duct 23 at substantially the same velocity at which they leave the picker teeth after separation from the pulp sheets 21, i.e., the fibres should maintain their velocity in both magnitude and direction from the point where they leave the picker teeth. More particularly, the velocity of the fibres separated from the pulp sheets 21 preferably does not change by more than about 20% in the duct 23. This is in contrast with other forming apparatus in which, due to flow separation, fibres do not travel in an ordered manner from the picker and, consequently, fibre velocities change as much as 100% or more during conveyance.

Further details of the picker device may be found in U.S. Specification No. 4,100,324. The particular differences between the apparatus shown in Figure 1 of the present specification and that of Figure 1 of U.S. Patent No. 4,100,324 is the means 27 for introducing particulate absorbent material into the melt blown fibre stream 18. The particle introduction means comprises a hopper 28 and air impeller 29 so arranged that the particles are ejected as a stream through a nozzle 17 into the fibre mat shortly after the nozzle 12 and whilst the melt blown fibres remain unset and tacky. The particles stick to the tacky fibres and are distributed throughout the fibre mat.

The fibres then cool as they continue in their path and/or they may be quenched with an air or water jet to aid cooling so that the fibres are set, with the particles adhered to them, before the fibres are formed into a web as described hereafter.

It is also possible to introduce the absorbent particles through the picker roll 20 and nozzle 23 either as an independent stream of particles or together with a stream of wood pulp fibres or a stream of staple textile fibres.

The hot air forming the melt blown fibres is at similar pressures and temperatures to that disclosed in U.S. Patent No. 4,100,324.

The set fibres and particles are condensed into a web by passing the mat of fibres between rolls 30 and 31 having foraminous surfaces that rotate continuously over a pair of fixed vacuum nozzles 32 and 33. As the integrated stream 18 enters the nip of the rolls 30 and 31, the carrying gas is sucked into the two vacuum nozzles 32 and 33 while the fibre blend is supported and slightly compressed by the opposed surfaces of the two rolls 30 and 31. This forms an integrated, self-supporting fibrous web 34 that has sufficient integrity to permit it to be withdrawn from the vacuum roll nip and conveyed to a wind-up roll 35.

Alternatively, the web may be formed on a moving wire screen. The web is then further processed and bonded by hot calendering, embossing or perforating, or by ultrasonic embossing.

Heated embossing rolls 36 and 37 are provided as more fully described in EP—A—0080383. These rolls are driven at different speeds and the consolidated fibre web is passed between the rolls to emboss the web and bond it. The differential speed of the rolls causes the relatively outer fibres to be in effect lifted or "brushed up" giving an enhanced thickness to the web.

The embossments on the roll may extend further from the roll surface than the thickness of the web which also aids in achieving an enhanced web product.

Fabrics made with the apparatus shown in figure 1 and with the apparatus shown but with the embossing head 40 and anvil roll 41 of U.S. Patent No. 4,100,324 replacing rolls 36 and 37 are shown in Figure 2, with the embossment indicated at 38 (see also Figure 3).

The primary feature of the invention is the inclusion of particulate material into the web. This is achieved by directing the particles through a nozzle into the stream of microfibres as they leave the die head, whilst the microfibres are still tacky and the particles adhere to the microfibres or even become partially embedded in them.

Improvements in the performance of the fabric in accordance with the invention is achievable by the use of surfactants such as described in British Patent No. 2,006,614. It is also possible to include fibrous material as disclosed in U.S. Patent No. 4,100,324 by the means disclosed therein.

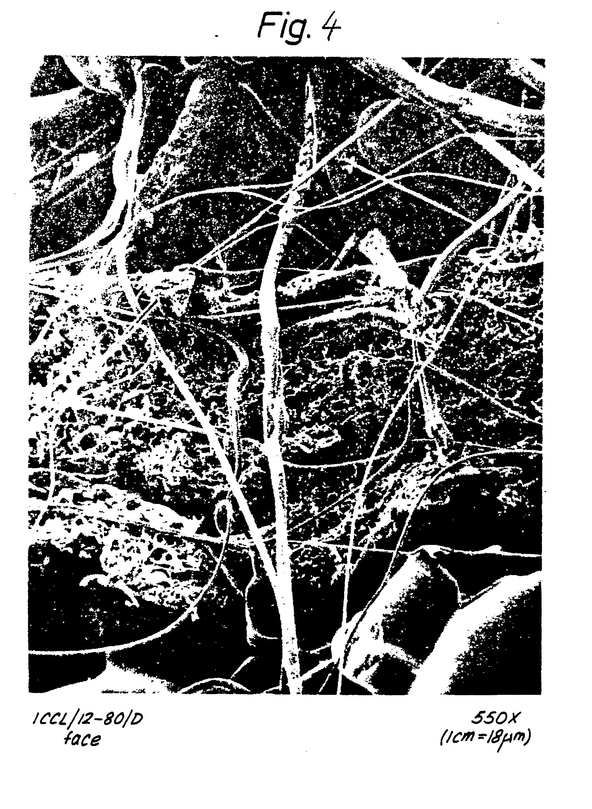

Granular organic materials may also be incorporated and particles of cellulose sponge have been used as illustrated in Figure 4. The water absorbent properties of the sponge contribute to the performance of the fabric as a water wipe. The sponge particle in Figure 4 has a dimension of about 0.16 mm in one direction.

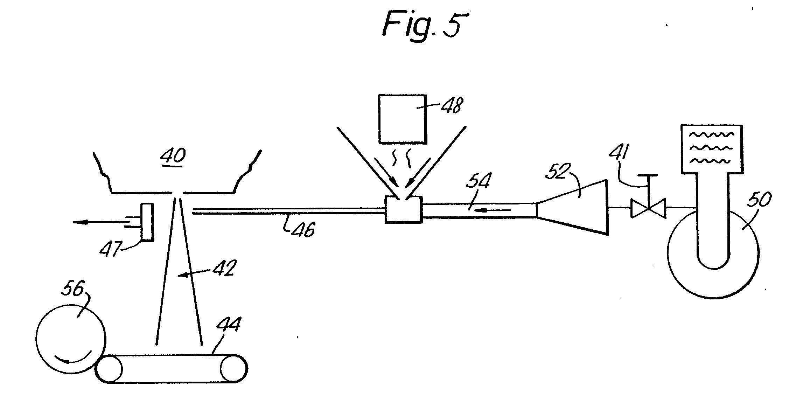

An alternative apparatus for use in producing a web in accordance with the invention and which is particularly suitable for the production of a web having particles of super absorbent material therein, as illustrated in Figure 5.

The melt blown fibres are produced by a device similar to that illustrated in Figure 1 and which is diagrammatically shown at 40 in Figure 5. The stream 42 of fibres passes downwardly towards a screen collector 44 on which the fibres are consolidated into a web.

Particles of super absorbent material are blown onto the mat of melt blown fibres through a nozzle 46 shortly after the fibres leave the outlet nozzle of the melt blown extruder apparatus 40. The air stream has a velocity of about 6,000 feet per minute (30 m/s). This speed is adjusted by valve 41 so that the majority of the particles are just trapped by the melt blown fibres and do not pass through to the dust catcher 47. The speed may be adjusted according to the weight and size of the particles and may vary from say about 4,000 to say 7,000 feet per minute (20 to 35 m/s) and dust is caught by a dust catcher 47.

The particulate super absorbent material is held in a particle dispenser 48 which may be that known as Model 500 made by the Oxi-Dry Corporation of Roselle, New Jersey, U.S.A., and is metered into an air stream formed by an air blower 50 passing through an air diffuser 52 and an air straightener 54. The powder in the dispenser is fed using an engraved metal roll in contact with two flexible blades. The cavity volume of the roll, roll speed and particle size control feed rate. An electrostatic charge is applied to the particles to promote individual particle separation in the composite, as gravity drops the particles into the air stream.

High turbulence at the conversion of the separate air streams, one containing fibre and the other particulate super absorbent, results in thorough mixing and a high capture percentage of the particulates by the microfibre. The particles are thus distributed substantially individually and spaced throughout the web formed from the fibre/particle mix by collecting it on the moving screen 44. It is then wound, as a nonwoven fabric, onto a roll 56.

As an example, polypropylene microfibre made from EXXON 3145 polypropylene resin from Exxon Chemical Company, Houston, Texas was prepared in accordance with the procedure generally known as melt blowing. It is described in article "Super Fine Thermoplastic Fibres", appearing in INDUSTRIAL AND ENGINEERING CHEMISTRY, Volume 48, No. 8, Pages 1342 to 1346 and in U.S. Patents Nos. 3,676,242 and 4,100,324.

A surfactant is applied to the microfibrous polypropylene at levels of 0.1 to 1.5 percent by weight to make the fibres wettable to aqueous solutions. In this Example, AEROSOL OT made by American Cyanamid Company, Wayne, New Jersey, was sprayed onto the fibres from dilute solution to an add on level of 0.27 percent by weight of fibre.

The powdered super absorbent used in this example was a synthetic organic polymer namely WATER-LOCK J—500 (i.e. a sodium polyacrylate polymer) made by Grain Processing Corporation, Muscatine, Iowa.

Four separate sample composites were made having 2.4, 5.1, 8.1 and 13 percent by composite weight respectively of super absorbent.

Several tests of the samples were prepared as indicated below.

The first test performed was a saturatedcapacity test. This test measures fluid holding capacity. Samples are cut, weighed, placed on a screen and submerged in a saline (0.85% Nacl) water bath for five minutes.

Saturated samples are removed from the bath and allowed to drain for two minutes. The sample is weighed and fluid weight absorbed is recorded as grams fluid per gram absorbent.

Fluid is then removed from the saturated sample using pressure applied with a vacuum box, a flexible rubber sheet and a screen support. Fluid retained after applying pressure for one minute is again measured by sample weights.

The second or horizontal wicking test measures distance of fluid migration as a function of time.

Sample strips 4 cm wide by 50 cm long were prepared and placed in a horizontal geometry on a plexiglass plate. A test fluid is put in a reservoir on one edge of the plate. One end of the samples are extended several centimetres off, the end of the plate and simultaneously pressed into the fluid. Distance wicked as a fraction of time is marked on the plate with ink and then recorded at the completion of each experiment.

A vertical wicking test on the samples was then performed. This test measures vertical distance wicked as a function of time.

Strips prepared as above are suspended vertically and the lower ends dipped into a fluid reservoir to a depth of 3.5 cm. Distance wicked above the fluid reservoir is recorded at specific time intervals.

Test results

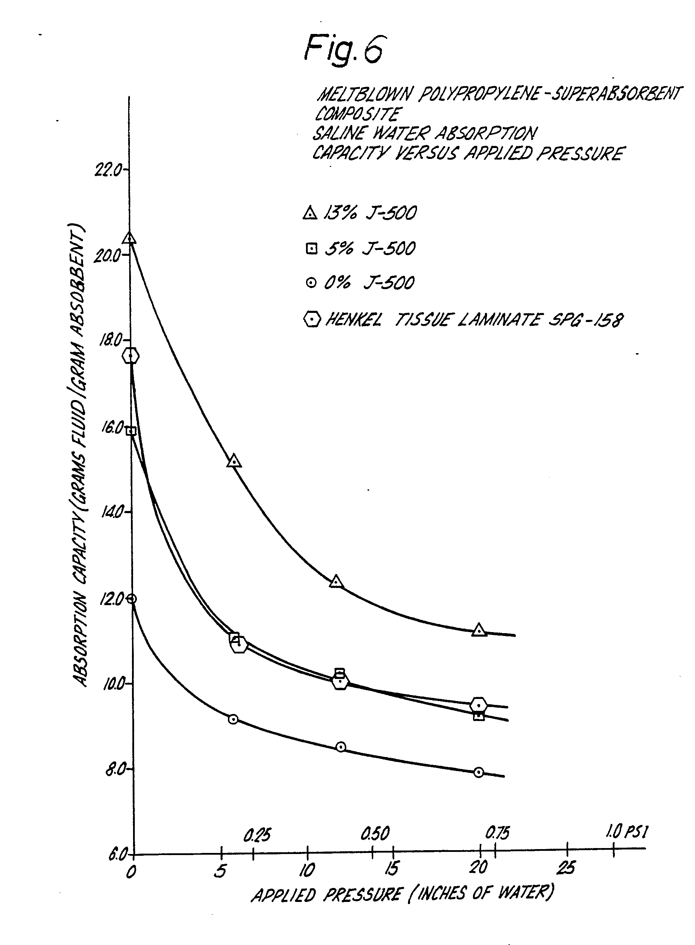

Saturated capacity of the microfibre super absorbent composites versus the microfibre control is shown in Figure 6. At zero pressure the control microfibre absorbs 12 grams fluid/gram absorbent. With 13 percent super absorbent in the microfibre the absorption capacity is 20.4 grams fluid per gram of absorbent (showing a 70 percent increase in capacity). The fluid used in this example is dilute saline solution (0.85 percent by weight).

Results

For comparison, a tissue laminate with super absorbent SPG 157 from Henkel Chemical Co., Minneapolis, Minnesota, U.S.A., is also shown in Figure 6. Results show the tissue laminate capacity to the 17.5 grams fluid per gram absorbent at zero pressure. The 13 percent J—500 composite demonstrates a 16.6 percent saturated capacity increase over the tissue laminate.

As pressure is applied all the materials release some fluid. The capacity advantage of the composite is maintained to pressures of at least 0.75 pounds per square inch ($5.1 \times 10^3$ N/m²).

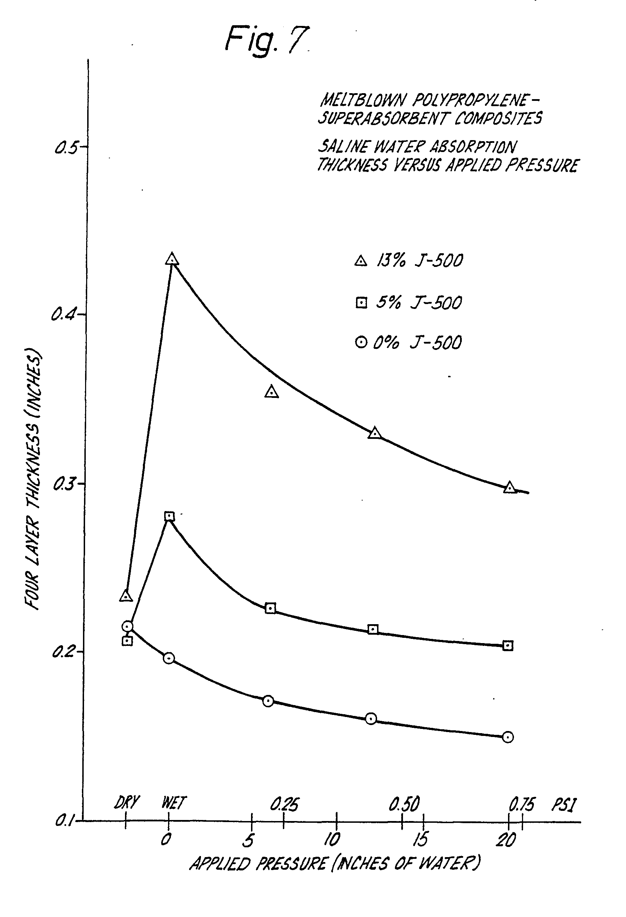

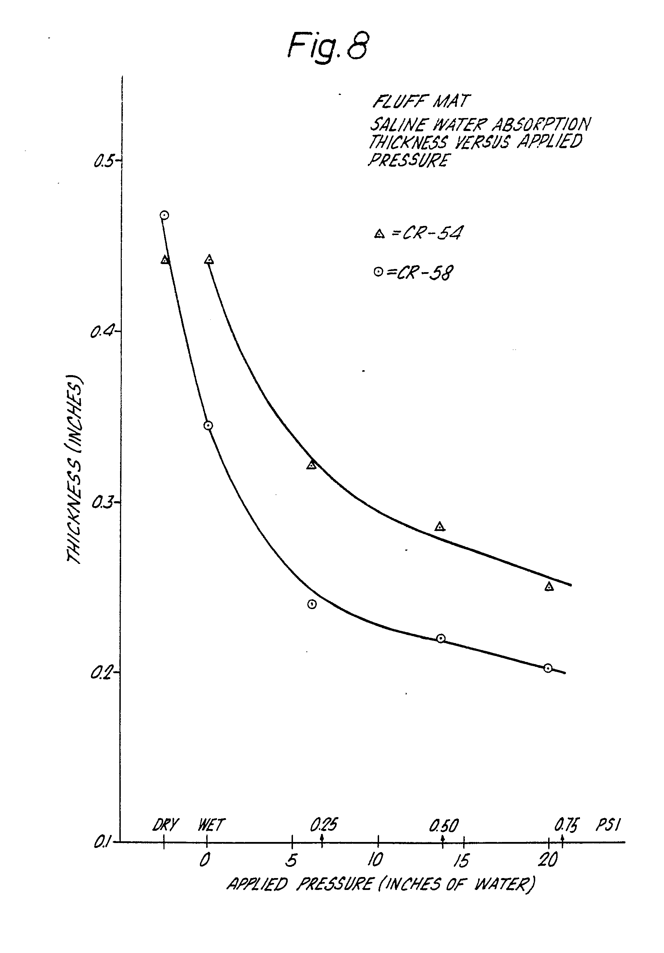

The volume (thickness) of the microfibre super absorbent composites is maintained better than microfibre and better than wood fluff absorbent (Figures 7 and 8). The super absorbent composites actually increase in volume as fluid is absorbed.

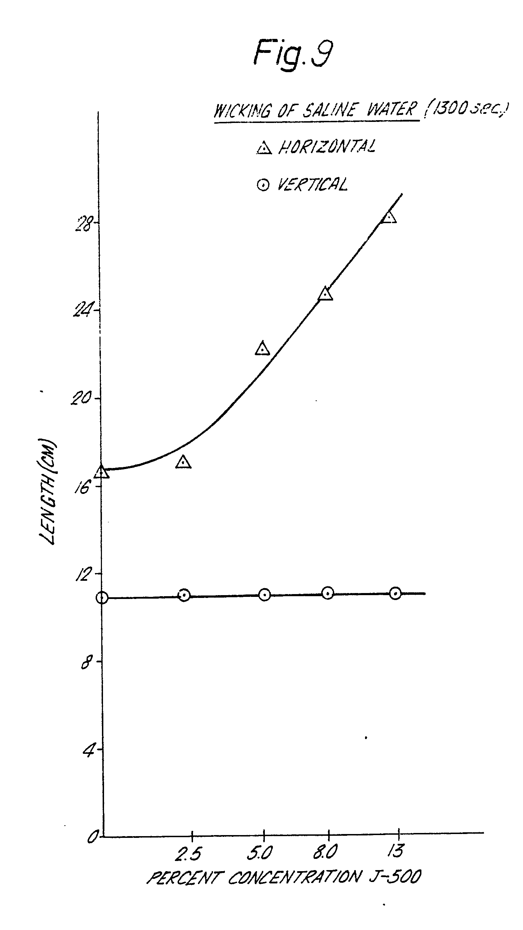

Horizontal wicking of the super absorbent composites versus an untreated microfibre control member (Figure 9) show that the composites have better fluid transfer rates. Comparing horizontal wicking of the 13 percent J—500 composite to the Henkel laminated tissue SPG 157 shows the composite to wick 18.8 cm after 600 seconds and the laminated tissue wicks 11.2 cm. This demonstrates a 67.9 percent increase in horizontal wicking for the composites over a commercially available product.

Vertical wicking shows that the superabsorbent composites have the same wicking properties as the microfibre control. No evidence of gel blocking is seen.

Comparing vertical wicking of 13 percent J—500 composite to the Henkel Laminated Tissue SPG—157 shows the composite to wick 11.1 cm, after 1300 seconds and the laminated tissue wicks 7.4 cm. This demonstrates a 50.0% increase in vertical wicking for the J—500 composite over a commercially available product.

Summary of Results

The microfibre composites containing Water-Lock J—500 absorbent shows improved absorbency characteristics of capacity and wicking over commercially available Henkel Laminated Tissue SPG—157 and also improvements over the currently produced microfibre without particle injected absorbents.

The particles of super absorbent material may have a relatively large diameter compared to the diameter of the individual microfibres and thus tend to be trapped within a network of the fibres and therefore little surface tack of the fibres is needed to maintain the super absorbent particles in place.

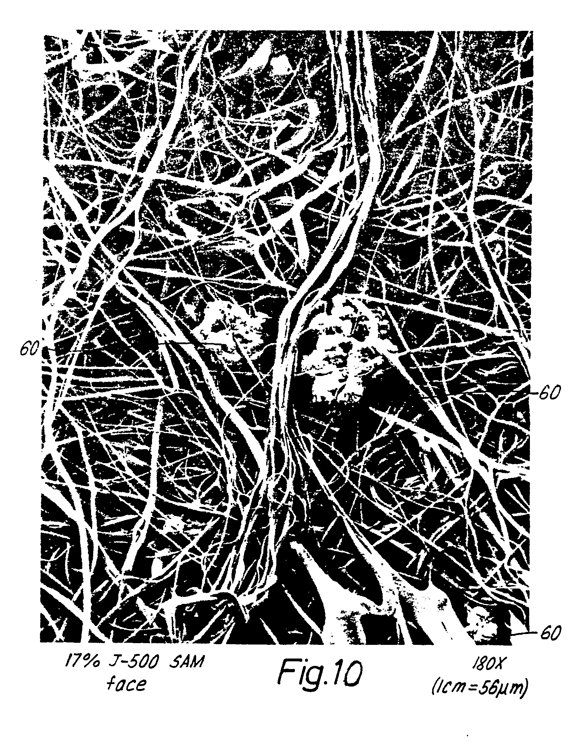

Figure 10 is an electron microscope photograph of one example of web in accordance with the invention including particles 60 of super absorbent material. The photographs are of a sample having 17% by weight of super absorbent to fibre material and are to a magnification of one hundred and eighty times. The maximum particles dimensions of the particles illustrated are between 122 × 139 microns and 168 × 213 microns and it can be seen that the particles are distributed substantially individually and spaced in the web sample.

**Claims**

1. A fluid retentive web comprising entangled meltblown polymer microfibres having an average fibre diameter greater than about 1 micron and absorbent material held within the web, characterised in that the material comprises particles of super absorbent (e.g. synthetic organic polymer, modified starch, cellulose, alginate or the like) distributed substantially individually and spaced throughout the web.

2. A web as claimed in claim 1 wherein the particles bear an electrostatically derived charge.

3. A web as claimed in either claim 1 or 2 wherein other fibres are mixed with the meltblown fibres.

4. A web as claimed in any preceding claim having wood pulp or staple textile fibres intermingled with the meltblown thermoplastic fibres.

5. A web as claimed in any preceding claim in which the fibres of the meltblown thermoplastic material comprise microfibres having an average fibre diameter less than about 50 microns.

6. A web as claimed in any preceding claim in which the blown fibres comprise microfibres averaging less than about 10 micrometers in diameter.

7. A web as claimed in any preceding claim in which the particles have a maximum dimension of between 1 and 100 microns.

8. A web as claimed in any preceding claim having a wetting agent therein.

9. A web as claimed in any preceding claim in which surfactant which assists in wetting of the web by a liquid to be absorbed is added to the web.

10. A sanitary napkin, diaper, incontinence pad or the like including a web containing substantially individual spaced particles of absorbent material as claimed in any preceding claim.

11. A method of making a fluid retentive non-woven web comprising extruding molten polymeric material in such a way as to produce a stream of meltblown polymeric microfibres having an average fibre diameter greater than 1 micron, directing absorbent material onto the stream of fibres, and then forming or consolidating the fibres into a web characterised in that particles of super absorbent (e.g. synthetic organic polymer, modified starch, cellulose, alginate or the like) are directed into the stream of meltblown fibres to be distributed substantially individually and spaced throughout the web, and in that an electrostatic charge is applied to the particles of super absorbent.

12. A method as claimed in claim 11 in which the particles are blown onto the stream of fibres shortly after the fibres leave an extrusion nozzle.

13. A method as claimed in either claim 11 or 12 in which the particles are injected into an air stream prior to the air stream impinging on the fibres.

14. A method as claimed in claim 13 in which the velocity of the air stream is adjusted so that the majority of the particles are trapped by the meltblown fibres and do not pass through the fibre stream.

15. A method as claimed in claim 14 in which the air stream has a velocity of about 6000 feet per minute (30 m/s).

16. A method as claimed in any one of claims 11 to 15 in which the web is hot calendered or embossed by passing it between heated patterned bonding rolls.

17. A method as claimed in claim 16 in which the depth of the embossing member on the patterned roll is greater than the thickness of the web.

18. A method as claimed in either claim 16 or 17 in which the embossing rolls are driven at different speeds.

19. A method as claimed in any of claims 11 to 18 in which other fibres are introduced into the stream of meltblown fibres prior to formation of the fibres into a web.

20. A method as claimed in any one of claims 11 to 19 in which a wetting agent is added to the meltblown fibres.

21. A method as claimed in any one of claims 11 to 20 wherein the electrostatic charge is applied prior to the particles contacting the fibres.

## Patentansprüche

1. Gewebe zum Aufsaugen von Flüssigkeiten, umfassend miteinander verflochtene schmelzgeblasene Polymermikrofasern mit einem durchschnittlichen Durchmesser der Fasern von mehr als etwa 1 Mikron sowie innerhalb des Gewebes angeordnetes Absorptionsmaterial, dadurch gekennzeichnet, daß das Material Teilchen aus Superabsorbenzien (zum Beispiel synthetische organische Polymere, modifizierte Stärke, Zellulose, Alginat o.ä.) umfaßt, die im wesentlichen einzeln und mit Abstand im Gewebe verteilt angeordnet sind.

2. Gewebe gemäß Anspruch 1, wobei die Teilchen eine elektrostatisch bedingte Aufladung aufweisen.

3. Gewebe gemäß Anspruch 1 oder 2, wobei andere Fasern mit den schmelzgeblasenen Fasern vermischt sind.

4. Gewebe gemäß irgendeinem der vorstehenden Ansprüche, wobei Zellulose- oder Textilstapelfasern den schmelzgeblasenen thermoplastischen Fasern beigemengt sind.

5. Gewebe gemäß irgendeinem der vorstehenden Ansprüche, wobei die Fasern aus schmelzgeblasenem thermoplastischen Material Mikrofasern mit einem durchschnittlichen Durchmesser der Fasern von weniger als etwa 50 Mikron umfassen.

6. Gewebe gemäß irgendeinem der vorstehenden Ansprüche, wobei die geblasenen Fasern Mikrofasern mit einem durchschnittlichen Durchmesser der Fasern von weniger als etwa 10 Mikrometer umfassen.

7. Gewebe gemäß irgendeinem der vorstehenden Ansprüche, wobei die Teilchen eine maximale, zwischen 1 und 100 Mikron liegende Abmessung aufweisen.

8. Gewebe gemäß irgendeinem der vorstehenden Ansprüche, wobei im Gewebe ein Netzmittel vorgesehen ist.

9. Gewebe gemäß irgendeinem der vorstehenden Ansprüche, wobei ein Tensid, das die Benetzung des Gewebes durch eine zu absorbierende Flüssigkeit unterstützt, dem Gewebe zugegeben ist.

10. Sanitärbinde, Winkel, Inkontinenzbinde o.ä., umfassend ein Gewebe, das im wesentlichen einzeln und mit Abstand angeordnete Teilchen aus Absorptionsmaterial gemäß irgendeinem der vorstehenden Ansprüche enthält.

11. Methode zur Herstellung von flüssigkeitsaufsaugendem Faservlies, umfassend das Extrudieren geschmolzener polymerer Materialien in der Weise, daß ein Strom aus schmelzgeblasenen polymeren Mikrofasern entsteht, die einen durchschnittlichen Durchmesser der Fasern von mehr als 1 Mikron aufweisen, wobei Absorptionsmaterial dem aus Fasern bestehenden Strom zugeführt wird, um anschließend die Fasern zu einem Gewebe zu formen oder in Gewebeform zusammenzuführen, dadurch gekennzeichnet, daß Teilchen aus Superabsorbenzien (zum Beispiel synthetische organische Polymere, modifizierte Stärke, Zellulose, Alginat o.ä.) dem Strom aus schmelzgeblasenen Fasern zugeführt werden, um so eine im wesentlichen einzelne und mit Abstand über das Gewebe verteilte Anordnung zu erreichen, und daß die Teilchen aus Superabsorbenzien elektrostatisch aufgeladen sind.

12. Methode gemäß Anspruch 11, wobei die Teilchen kurz nach dem Austritt der Fasern aus der Extrusionsdüse auf den aus Fasern bestehenden Strom aufgeblasen werden.

13. Methode gemäß Anspruch 11 oder 12, wobei die Teilchen in einen Luftstrom eingeblasen werden, bevor der Luftstrom auf die Fasern auftrifft.

14. Methode gemäß Anspruch 13, wobei die Geschwindigkeit des Luftstroms so eingestellt ist, daß die Mehrzahl der Teilchen von den schmelzgeblasenen Fasern eingefangen wird und nicht den aus Fasern bestehenden Strom passiert.

15. Methode gemäß Anspruch 14, wobei der Luftstrom eine Geschwindigkeit von etwa 6.000 Fuß/Minute (30 m/s) aufweist.

16. Methode gemäß irgendeinem der Ansprüche 11 bis 15, wobei das Gewebe dadurch heißkalandriert oder geprägt wird, das es zwischen beheizten, Muster aufweisenden Klebewalzen durchgeführt wird.

17. Methode gemäß Anspruch 16, wobei die Tiefe des Prägeelements auf der mit Muster versehenen Walze größer als die Gewebedicke ist.

18. Methode gemäß Anspruch 16 oder 17, wobei die Prägewalzen mit unterschiedlichen Drehzahlen betrieben werden.

19. Methode gemäß irgendeinem der Ansprüche 11 bis 18, wobei dem Strom aus schmelzgeblasenen Fasern andere Fasern zugegeben werden, bevor die Fasern ein Gewebe bilden.

20. Methode gemäß irgendeinem der Ansprüche 11 bis 19, wobei den schmelzgeblasenen Fasern ein Netzmittel zugegeben wird.

21. Methode gemäß irgendeinem der Ansprüche 11 bis 20, wobei die elektrostatische Aufladung erfolgt, bevor die Teilchen mit den Fasern in Berührung kommen.

**Revendications**

1. Une feuille continue pour la rétention des fluides, comprenant des microfibres de polymère soufflées en fusion, entrecroisées, ayant un diamètre moyen de fibre supérieur à environ 1 micron et un matériau absorbant retenu à l'intérieur de la feuille continue, caractérisée en ce que le matériau comprend des particules de superabsorbant (comme par exemple un polymère organique de synthèse, de l'amidon modifié, de la cellulose, un alginate ou similaire) réparties sensiblement individuellement et dispersées dans toute la feuille.

2. Une feuille selon la revendication 1, dans laquelle les particules portent une charge d'origine électrostatique.

3. Une feuille selon l'une ou l'autre des revendications 1 ou 2, dans laquelle d'autres fibres sont mélangée aux fibres soufflées en fusion.

4. Une feuille selon l'une quelconque des revendications précédentes, contenant de la pâte de bois ou des fibres textiles discontinues, mélangées intimement aux fibres thermoplastiques soufflées en fusion.

5. Une feuille selon l'une quelconque des revendications précédentes, dans laquelle les fibres de matériau thermoplastique soufflé en fusion comprennent des microfibres ayant un diamètre moyen de fibre inférieur à environ 50 microns.

6. Une feuille selon l'une quelconque des revendications précédentes, dans laquelle les fibres soufflées comprennent des microfibres ayant en moyenne moins d'environ 10 micromètres de diamètre.

7. Une feuille selon l'une quelconque des revendications précédentes, dans laquelle les particules ont une dimension maximale comprise entre 1 et 100 microns.

8. Une feuille selon l'une quelconque des revendications précédentes, contenant un agent de mouillage à l'intérieur de celle-ci.

9. Une feuille selon l'une quelconque des revendications précédentes, dans laquelle on ajoute à la feuille un agent tensio-actif qui favorise le mouillage de la feuille par un liquide à absorber.

10. Une serviette hygiénique, une couche, une garniture d'incontinence ou similaire, comprenant une feuille contenant des particules de matériau absorbant sensiblement individuelles et dispersées selon l'une quelconque des revendications précédentes.

11. Un procédé de fabrication d'une feuille non tissée pour la rétention des fluides comprenant l'extrusion d'un matériau polymère fondu de manière telle que l'on obtient un courant de microfibres polymères soufflées en fusion ayant un diamètre moyen de fibres supérieur à 1 micron, l'acheminement de matériau absorbant sur le courant de fibres, et ensuite la mise en forme ou la consolidation des fibres en une feuille, caractérisé en ce que des particules de superabsorbant (comme par exemple un polymère organique de synthèse, de l'amidon modifié, de la cellulose, un alginate ou similaire) sont acheminées dans le courant de fibres soufflées en fusion pour être réparties sensiblement individuellement et dispersées dans toute la feuille, et en ce que l'on applique une charge électrostatique aux particules de superabsorbant.

12. Un procédé selon la revendication 11, dans lequel les particules sont soufflées sur le courant de fibres peu de temps après que les fibres aient quitté une buse d'extrusion.

13. Un procédé selon l'une quelconque des revendications 11 ou 12, dans lequel les particules sont injectées dans un courant d'air avant que le courant d'air ne heurte les fibres.

14. Un procédé selon la revendication 11, dans lequel o règle la vélocité du courant d'air de manière que la majorité des particules soient piégées par les fibres soufflées en fusion et ne passent pas à travers le courant de fibres.

15. Un procédé selon la revendication 14 dans lequel, le courant d'air a une vélocité d'environ 30 mètres par seconde (6.000 pieds/mn).

16. Un procédé selon l'une quelconque des revendications 11 à 15, dans lequel la feuille est calandrée ou grainée à chaud en la faisant passer entre des gaufroirs de collage chauffés.

17. Un procédé selon la revendication 16, dans lequel la profondeur de l'organe de gaufrage sur le gaufroir est supérieure à l'épaisseur de la feuille.

18. Un procédé selon l'une quelconque des revendications 16 ou 17, dans lequel les gaufroirs sont entraînés à des vitesses différentes.

19. Un procédé selon l'une quelconque des revendications 11 à 18, dans lequel on introduit d'autres fibres dan le courant de fibres soufflées en fusion avant la mise en forme des fibres en une feuille continue.

20. Un procédé selon l'une quelconque des revendications 11 à 19, dans lequel on ajoute un agent mouillant aux fibres soufflées en fusion.

21. Un procédé selon l'une quelconque des revendications 11 à 20, dans lequel la charge électrostatique est appliquée avant que les particules viennent au contact des fibres.

Fig.1.

Fig.2.

Fig.3.

# Fig. 4

ICCL/12-80/D
face

550X
(1cm=18μm)

Fig. 5

# Fig.6

*MELTBLOWN POLYPROPYLENE-SUPERABSORBENT*
*COMPOSITE*
*SALINE WATER ABSORPTION*
*CAPACITY VERSUS APPLIED PRESSURE*

△ *13% J-500*

▣ *5% J-500*

⊙ *0% J-500*

⬡ *HENKEL TISSUE LAMINATE SPG-158*

4

# Fig. 7

MELTBLOWN POLYPROPYLENE-
SUPERABSORBENT COMPOSITES

SALINE WATER ABSORPTION
THICKNESS VERSUS APPLIED PRESSURE

△ 13% J-500

▫ 5% J-500

⊙ 0% J-500

FOUR LAYER THICKNESS (INCHES)

0.5

0.4

0.3

0.2

0.1

DRY   WET        0.25            0.50            0.75   PSI

0        5        10        15        20

APPLIED PRESSURE (INCHES OF WATER)

# Fig.8

FLUFF MAT
SALINE WATER ABSORPTION
THICKNESS VERSUS APPLIED
PRESSURE

△ =CR-54

⊙ =CR-58

THICKNESS (INCHES)

APPLIED PRESSURE (INCHES OF WATER)

# Fig.9

WICKING OF SALINE WATER (1300 sec.)

△ HORIZONTAL

⊙ VERTICAL

17% J-500 SAM
face

Fig.10

180X
(1cm=56μm)